(19) Europäisches Patentamt
European Patent Office
Office européen des brevets

(11) Veröffentlichungsnummer: **0 186 026**
**B1**

(12) # EUROPÄISCHE PATENTSCHRIFT

(45) Veröffentlichungstag der Patentschrift: **19.04.89**

(21) Anmeldenummer: **85115633.1**

(22) Anmeldetag: **09.12.85**

(51) Int. Cl.[4]: **C 07 D 285/00**, C 07 D 513/04, C 11 B 9/00, A 23 L 1/00 // (C07D513/04, 285:00, 209:00)

(54) **Unsymmetrische Dihydro-dithiazine, Verfahren zu ihrer Herstellung und ihre Verwendung als Riech- und Geschmacksstoffe.**

(30) Priorität: **22.12.84 DE 3447209**

(43) Veröffentlichungstag der Anmeldung: **02.07.86 Patentblatt 86/27**

(45) Bekanntmachung des Hinweises auf die Patenterteilung: **19.04.89 Patentblatt 89/16**

(84) Benannte Vertragsstaaten: **AT CH DE FR GB IT LI NL SE**

(56) Entgegenhaltungen:
**GB-A-1 364 747**

**CHEMICAL ABSTRACTS, Band 98, Nr. 11, 14 März 1983, Seite 428, Nr. 87793f, Columbus, Ohio, US; K. KUBOTA et al.: "Comparison of the composition of sulfur-containing compounds in the odors of cooked E. superba (Antarctic krill) and S. lucens (Sakura-ebi)" & NIPPON NOGEI KAGAKU KAISHI 1982, 56(11), 1049-52**

**Die Akte enthält technische Angaben, die nach dem Eingang der Anmeldung eingereicht wurden und die nicht in dieser Patentschrift enthalten sind.**

(73) Patentinhaber: **Haarmann & Reimer GmbH, Postfach 1253, D-3450 Holzminden (DE)**

(72) Erfinder: **Brüning, Jürgen, Dr., Sparenbergstrasse 39, D-3450 Holzminden (DE)**
Erfinder: **Emberger, Roland, Dr., Bärenfang 4, D-3450 Holzminden (DE)**
Erfinder: **Hopp, Rudolf, Dr., Auf dem Gehrenkamp 28, D-3450 Holzminden (DE)**
Erfinder: **Köpsel, Manfred, Dr., Schinkelstrasse 1, D-3450 Holzminden (DE)**
Erfinder: **Sand, Theodor, Dr., Vogelsang 3, D-3450 Holzminden (DE)**
Erfinder: **Werkhoff, Peter, Dr., Sieplerstrasse 24, D-3470 Höxter 1 (DE)**

(74) Vertreter: **Schumacher, Günter, Dr., c/o Bayer AG Konzernverwaltung RP Patentabteilung, D-5090 Leverkusen 1 Bayerwerk (DE)**

**EP 0 186 026 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue unsymmetrische mono- oder bicyclische Dihydro-dithiazine, ein Verfahren zu ihrer Herstellung und ihre Verwendung als Riech- und Geschmackstoffe.

Dihydro-dithiazine und ihre Verwendung als Riech- und Geschmacksstoffe sind bekannt. In der GB-PS-1 364 747 ist allgemein die Verwendung symmetrischer Dihydrodithiazine, in den US-Patentschriften 4 200 741, 4 200 742 und 4 228 278 speziell die Verwendung des symmetrischen 2,4,6-Triisobutyl-dihydro-1,3,5-dithiazins als Geruchs- und Geschmackstoff beschrieben. Aromastärke und Aromacharakter dieser bekannten Dihydro-dithiazine befriedigen jedoch in der Praxis noch nicht.

Ferner wird in Nippon Nogei Kagaku Kaishi 1982, 56 (11), 1049 - 52 (Ref. CA 98 (1983) 87793f) beschrieben, daß neben Trithiolanen, Dithiinen und symmetrischen Dihydro-1,3,5-dithiazinen, wie dem 2,4,6-Trimethyl-dihydro-3,5-dithiazin, auch die unsymmetrischen Dihydro-1,3,5-dithiazine 4-ethyl-2,6-dimethyl- und 2-ethyl-4,6-dimethyl-dihydro-dithiazine, gaschromatographisch im Geruch bestimmter Meerestiere nachgewiesen werden konnten.

Überraschenderweise wurde gefunden, daß bestimmte unsymmetrische Dihydro-dithiazine ein wesentlich stärkeres Aroma aufweisen und daß vor allem in ihrem Aromacharakter die angestrebte Röst- und Nuß-note wesentlich stärker ausgeprägt ist. Im Gegensatz zu den bekannten Dihydro-dithiazinen tritt bei den unsymmetrischen Dihydro-dithiazinen auch keine in Nußaromen unerwünschte speckige Note in Erscheinung.

Die Erfindung betrifft daher neue unsymmetrische mono- oder bicyclische Dihydro-dithiazine der Formel

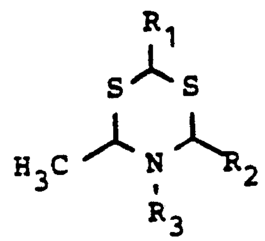

(I)

in der

entweder jeweils einer der Substituenten $R_1$ und $R_2$ für eine $C_3$-$C_5$-Alkylgruppe steht während der andere Substituent eine Methylgruppe ist und $R_3$ für ein Wasserstoffatom steht,

oder $R_1$ für eine Methylgruppe steht und $R_2$ und $R_3$ zusammen einen $C_3$-$C_4$-Alkylenrest bilden.

Als $C_3$-$C_5$-Alkylreste kommen vor allem der n-Propyl-, Isopropyl-, n-Butyl-, Isobutyl-, 2-Butyl-, n-Pentyl- und der Isopentylrest in Betracht.

Als $C_3$-$C_4$-Alkylenreste seien vor allem der Propylen-1,3 und der Butylen-1,4-Rest genannt.

Als bevorzugte Vertreter der erfindungsgemäßen unsymmetrischen Dihydro-dithiazine seien genannt:

4.6-Dimethyl-2-isopropyl-dihydro-1.3.5-dithiazin,
2.4-Dimethyl-6-isopropyl-dihydro-1.3.5-dithiazin,
4.6-Dimethyl-2-isobutyl-dihydro-1.3.5-dithiazin,
2.4-Dimethyl-6-isobutyl-dihydro-1.3.5-dithiazin,
4.6-Dimethyl-2-(2-butyl)-dihydro-1.3.5-dithiazin,
2.4-Dimethyl-6-(2-butyl)-dihydro-1.3.5-dithiazin,
4.6-Dimethyl-2-n-propyl-dihydro-1.3.5-dithiazin,
2.4-Dimethyl-6-n-propyl-dihydro-1.3.5-dithiazin und
2.4-Dimethyl-tetrahydro-pyrrolo-[2.1-d]-[1.3.5]-dithiazin.

Die Erfindung betrifft ferner ein Verfahren zur Herstellung der unsymmetrischen Dihydro-dithiazine der Formel (I); das Verfahren ist dadurch gekennzeichnet, daß man zur Herstellung der monocyclischen Verbindungen ein Gemisch aus Acetaldehyd und einem $C_4$-$C_6$-Alkanal mit Ammoniak reagieren läßt und das Reaktionsprodukt mit Schwefelwasserstoff umsetzt. Zur Herstellung der bicyclischen Verbindungen läßt man Acetaldehyd mit 1-Pyrrolin oder 2.3.4.5-Tetrahydropyridin reagieren und setzt das Reaktionsprodukt mit Schwefelwasserstoff um.

Außerdem betrifft die Erfindung die Verwendung der unsymmetrischen Dihydro-dithiazine der Formel (I) als Riech- und Geschmackstoffe.

Die Herstellung der monocyclischen unsymmetrischen Dihydro-dithiazine kann im Prinzip nach dem bereits von Wöhler und Liebig in Ann. 61, 1 (1847) beschriebenen Verfahren zur Herstellung von Thialdin (2.4.6-Trimethyldihydro-1.3.5-dithiazin) erfolgen. In Analogie zu diesem Verfahren wird ein Gemisch aus Acetaldehyd und $C_4$-$C_6$-Alkanal, in dem beide Komponenten in einem Molverhältnis von 2,2 bis 1,8 : 1, vorzugsweise 2 : 1 vorliegen, bei 0 bis 20°C mit einem 2,5- bis 4-molaren Überschuß, bezogen auf die Aldehyd-Gruppen, an wäßrigem, konzentriertem Ammoniak versetzt. Nach 0,5- bis 1-stündigem Rühren bei Raumtemperatur wird in das Reaktionsgemisch bis zur Sättigung Schwefelwasserstoff eingeleitet. Anschließend wird das Reaktionsgemisch 12 bis 24 Stunden bei Raumtemperatur aufbewahrt. Nach üblicher Aufarbeitung und fraktionierter Destillation erhält man ein Gemisch der Isomeren 2.4-Dimethyl-6-$C_3$-$C_5$-alkyl- und 4.6-Dimethyl-2-$C_3$-$C_5$-alkyl-dihydro-1.3.5-dithiazine. Das Isomerengemisch kann durch übliche Trennverfahren, z. B. präparative Säulenchromatographie an Kieselgel, in die einzelnen Komponenten aufgeteilt werden. Da die

Isomerengemische in ihren sensorischen Eigenschaften aber nur unerheblich von den sensorischen Eigenschaften der isolierten Isomeren abweichen, kann man im allgemeinen auf die Trennung der Isomeren verzichten und das Isomerengemisch als solches als Riech- bzw. Geschmackstoff verwenden.

Zur Herstellung der bicyclischen unsymmetrischen Dihydro-dithiazine der Formel (I) wird Acetaldehyd bei Raumtemperatur tropfenweise mit der etherischen Lösung von 1-Pyrrolin oder 3.4.5.6-Tetrahydropyridin versetzt. Das Molverhältnis Acetaldehyd : 1-Pyrrolin bzw. 3.4.5.6-Tetrahydropyridin beträgt 1.8 bis 2,2 : 1, vorzugsweise 2 : 1. Das Reaktionsgemisch wird bei einer Temperatur von 0 bis 20°C mit Schwefelwasserstoff gesättigt und anschließend 12 bis 24 Stunden bei Raumtemperatur aufbewahrt.

Die erfindungsgemäßen unsymmetrischen Dihydro-dithiazine der Formel (I) sind wertvolle Riech- und Geschmackstoffe, die wegen ihres nußartigen Geruchs und Geschmacks dazu dienen, den Charakter von Riech- und Geschmackstoffkompositionen zu verändern, zu verbessern und zu verstärken. Bei der Verwendung als Riechstoff werden die erfindungsgemäßen Verbindungen in Kombinationen mit anderen, an sich bekannten Riechstoffen (Arctander, Perfume and Flavor Chemicals, Montclair, N.J. (USA), 1969) und etherischen Ölen (Arctander, Perfume and Flavor Materials of Natural Origin., Elisabeth, N.J. (USA), 1960) angewandt und führen zu Parfümbasen und Riechstoffkompositionen mit ausdrucksstarken Noten, die sich hervorragend für die Parfümierung von Fertigprodukten des Aerosol-, Waschmittel- und des chemisch-technischen Sektors, insbesondere aber des Feinparfümerie- oder Kosmetiksektors eignen, z. B. für Detergentien, Haarpflegemittel, Schaumbäder, Badesalz, Geschirrspülmittel, Waschpulver, Seifen, Antiperspirans, Puder, Cremes, Rasierwasser, After-Shave-Lotions, Raumluftverbesserer, WC-Reiniger, Raum-Sprays, Antiperspirans-Sprays, Deodorant-Sprays, Körper-Sprays, Insektizid-Sprays und Sonnenschutzmittel.

Die Herstellung der Parfümkompositionen und parfümierten Produkte erfolgt in üblicher Weise, beispielsweise durch Zusammengeben der Komponenten.

Weiterhin sind die erfindungsgemäßen Verbindungen wertvolle Geschmacksstoffe, die sich durch sehr niedrige Geschmacksschwellenwerte auszeichnen.

So liegt in wäßriger, 0,5-%-iger Kochsalzlösung die Erkennungsschwelle zwischen 0,5 und 5 x $10^{-3}$ ppm.

Bei der und über der Erkennungsschwelle lautet die Geschmacksbeschreibung einiger beispielhaften Gemische bzw. Substanzen wie folgt:

Gemisch A (Gemisch aus 4.6-Dimethyl-2-isopropyl-dihydro-1.3.5-dithiazin und 2.4-Dimethyl-6-isopropyl-dihydro-1.3.5-dithiazin, hergestellt nach Beispiel 4): Erdnuß, Kakao, Röstnote

Gemisch B (Gemisch aus 4.6-Dimethyl-2-isobutyl-dihydro-1.3.5-dithiazin und 2.4-Dimethyl-6-isobutyl-dihydro-1.3.5-dithiazin, hergestellt nach Beispiel 1): Erdnuß, Haselnuß, Röstnote, Fleisch

Gemisch C (Gemisch aus 4.6-Dimethyl-2-(2-butyl)-dihydro-1.3.5-dithiazin und 2.4-Dimethyl-6-(2-butyl)-dihydro-1.3.5-dithiazin, hergestellt nach Beispiel 2): Erdnuß, Röstnote, fettig

Substanz D (2.4-Dimethyltetrahydropyrrolo[2.1-d]-[1.3.5]-dithiazin): Erdnuß, Zwiebel, Röstnote

Neben ihrer spezifischen Charakterisierung in Richtung Hasel- bzw. Erdnuß in entsprechenden Aromakompositionen wirken die erfindungsgemäßen Verbindungen in allen Nicht-Nußtypen insbesondere abrundend und mehr Natürlichkeit vermittelnd.

Die unter Verwendung der erfindungsgemäßen Verbindungen hergestellten Aromakompositionen können im gesamten Nahrungsmittel- und Genußmittelbereich, Mundpflege und Tierfutter eingesetzt werden. Insbesondere sind sie geeignet für Fondantmassen, Nougatmassen, Fettmassen, Margarine, Speiseöl, Kuchenmehle, Biskuitmassen, Backwaren, Extrusionsprodukte, Milchprodukte, Sauermilchprodukte, Getränke, Speiseeis, Gummi, Mundpflegemittel, Tabakprodukte, Fertiggerichte, Fleisch- und Wurstprodukte, Suppen, Soßen, Gemüsekonserven, Spirituosen, pflanzliche und mikrobielle Proteine und alle Arten von industriell gefertigtem Tierfutter.

Die erfindungsgemäßen Dihydrodithiazine werden in Mengen von 0,5 x $10^{-3}$ ppm bis 1 %, vorzugsweise 1 x $10^{-3}$ ppm bis 100 ppm, bezogen auf das verzehrsfertige Nahrungsmittel verwendet.

**Beispiel 1**

Das Gemisch aus 48,1 g (1,1 Mol) Acetaldehyd und 47 g (0,55 Mol) Isovaleraldehyd wird bei 5°C mit 95,2 g (1,93 Mol) konzentriertem, wäßrigen Ammoniak versetzt. Nach der Zugabe des Ammoniaks wird eine halbe Stunde bei Raumtemperatur gerührt und anschließend 3 Stunden bis zur Sättigung Schwefelwasserstoff eingeleitet. Der ausgefallene Niederschlag wird durch Zugabe von 150 ml Wasser gelöst und das Reaktionsgemisch 15 Stunden bei Raumtemperatur aufgewahrt.

Zur Aufarbeitung wird die organische Phase abgetrennt, die wäßrige Phase mit Cyclohexan extrahiert und die Cyclohexan-Phase mit der organischen Phase vereinigt. Die organische Phase wird neutralgewaschen, getrocknet und im Vakuum vom Lösungsmittel befreit. Der Rückstand wird einer fraktionierten Destillation unterworfen. Die zwischen 104 und 115°C bei 2,5 mbar übergehende Fraktion enthält das gewünschte Gemisch der beiden Dimethylisobutyl-dihydro-dithiazine. Ein Teil dieses Gemisches wird unmittelbar als Zusatz zu einer Aromakomposition verwendet.

Der andere Teil wird durch chromatographische Trennung an Kieselgel 60 unter Verwendung des Lösungsmittelgemisches Cyclohexan/Essigester (80 : 20) in die reinen Komponenten 4.6-Dimethyl-2-isobutyldihydro-1.3.5-dithiazin und 2.4-Dimethyl-6-isobutyldihydro-1.3.5-dithiazin aufgetrennt. Die reinen Komponenten wurden durch ihre Massenspektren charakterisiert.

Massenspektrum des 4.6-Dimethyl-2-isobutyldihydro-1.3.5-dithiazins: 44 (100); 71 (34); 70 (27); 41 (27); 60 (26); 43 (25); 45 (23); 59 (21).

Massenspektrum des 2,4-Dimethyl-6-isobutyldihydro-1,3,5-dithiazins: 43 (100); 44 (83); 60 (69); 86 (67); 59 (61); 70 (56); 41 (51); 45 (46).

**Beispiel 2**

63 g (1,43 Mol) Acetaldehyd, 61,6 g (0,71 Mol) 2-Methyl-butyraldehyd und 124 g (2,5 Mol) konzentriertes, wäßriges Ammoniak werden wie in Beispiel 1 beschrieben umgesetzt.

Bei der fraktionierten Destillation geht das Isomerengemisch zwischen 72° und 135°C bei 3 mbar über. Ein Teil dieses Gemisches wird unmittelbar als Zusatz zu einer Aromakomposition verwendet.

Der andere Teil durch Säulenchromatographie in die reinen Komponenten, 4.6-Dimethyl-2-(2-butyl)-dihydro-1.3.5-dithiazin und 2.4-Dimethyl-6-(2-butyl)-dihydro-1.3.5-dithiazin aufgetrennt. Die reinen Komponenten wurden durch ihre Massenspektren charakterisiert.

Massenspektrum des 4.6-Dimethyl-2-(2-butyl)-dihydro-1.3.5-dithiazins: 44 (100); 71 (56); 70 (43); 205 (32); 103 (31); 41 (14); 56 (11); 45 (11).

Massenspektrum des 2.4-Dimethyl-6-(2-butyl)-dihydro-1.3.5-dithiazins: 86 (100); 44 (82); 205 (50); 112 (37); 84 (29); 60 (27); 41 (24); 145 (24).

**Beispiel 3**

48,1 g (1,1 Mol) Acetaldehyd, 40 g (0,55 Mol) Butyraldehyd und 95,2 g (1,93 Mol) konzentriertes wäßriges Ammoniak werden wie in Beispiel 1 beschrieben umgesetzt.

Bei der fraktionierten Destillation geht ein Gemisch der beiden Isomeren Dimethyl-n-propyldihydrodithiazine zwischen 55 und 120°C bei 2,5 mbar über. Ein Teil dieses Gemisches wird unmittelbar als Zusatz zu einer Aromakomposition verwendet.

Der andere Teil wird durch Säulenchromatographie in seine beiden Komponenten, 4.6-Dimethyl-2-n-propyldihydro-1.3.5-dithiazin und 2.4-Dimethyl-6-n-propyldihydro-1.3.5-dithiazin aufgetrennt. Die reinen Komponenten wurden durch ihre Massenspektren charakterisiert.

Massenspektrum des 4.6-Dimethyl-2-n-propyldihydro-1.3.5-dithiazins: 44 (100); 71 (29); 70 (23); 191 (12); 103 (12); 60 (11); 56 (11); 45 (11).

Massenspektrum des 2.4-Dimethyl-6-n-propyldihydro-1.3.5-dithiazins: 44 (100); 72 (92); 98 (51); 70 (40); 60 (31); 191 (30); 59 (27); 27 (25).

**Beispiel 4**

48,1 g (1,1 Mol) Acetaldehyd, 40 g (0,55 Mol) 2-Methylpropionaldehyd und 95,2 g (1,93 Mol) konzentriertes, wäßriges Ammoniak werden wie in Beispiel 1 beschrieben umgesetzt.

Bei der fraktionierten Destillation geht ein Gemisch der beiden Isomeren Dimethyl-isopropyl-dihydrodithiazinen bei 64 bis 87°C und 1,7 mbar über. Ein Teil dieses Gemisches wird unmittelbar als Zusatz zu einer Aromakomposition verwendet.

Der andere Teil wird durch Säulenchromatographie an Kieselgel 60 in seine beiden Komponenten, 4.6-Dimethyl-2-isopropyl-dihydro-1.3.5-dithiazin und 2.4-Dimethyl-6-isopropyl-dihydro-1.3.5-dithiazin, aufgetrennt.

Die reinen Komponenten wurden durch ihre Massenspektren charakterisiert.

Massenspektrum des 4.6-Dimethyl-2-isopropyl-dihydro-1.3.5-dithiazins: 44 (100); 71 (38); 70 (29); 191 (27); 103 (19); 56 (8); 55 (8); 45 (7).

Massenspektrum des 2.4-Dimethyl-6-isopropyl-dihydro-1.3.5-dithiazins: 44 (100); 72 (93); 191 (54); 98 (26); 55 (24); 84 (23); 60 (20); 99 (19).

**Beispiel 5**

Zu der Lösung von 34,5 g (0,5 Mol) 1-Pyrrolin in 150 ml Ether werden 44 g (1 Mol) Acetaldehyd zugetropft. Anschließend wird unter Kühlen mit Eis in die Reaktionsmischung Schwefelwasserstoff bis zur Sättigung eingeleitet. Nach Zugabe von 10 g Magnesiumsulfat wird das Reaktionsgemisch 15 Stunden bei Raumtemperatur aufbewahrt. Nach dem Abdestillieren des Ethers wird der Rückstand fraktioniert destilliert. Es

werden 43 g 2,4-Dimethyltetrahydro-pyrrolo[2.1-d]-[1.3.5]-dithiazin (Kp. bei 6 mbar: 105° C) erhalten.

**Beispiel 6**

Eine Aromakomposition A mit Haselnußaroma wird durch Mischen folgender Bestandteile hergestellt:

| | |
|---|---|
| Vanillin | 30 |
| Benzaldehyd | 10 |
| Furfural | 5 |
| 2-Ethyl-3,4(3,6)-dimethylpyrazin | 5 |
| 2-Methyl-3-ethylpyrazin | 5 |
| Resorcindimethylether | 50 |
| Propylenglykol | 895 |
| Gewichtsteile | 1000 |

Eine Aromakomposition B wird durch Zugabe von 10 Gewichtsteilen des Isomerengemisches 2.4(4.6)-Dimethyl-6(2)-isopropyl-dihydro-1.3.5-dithiazin, erhalten nach Beispiel 4, zu A hergestellt.

Eine Aromakomposition C wird durch Zugabe von 5 Gewichtsteilen des Isomerengemisches 2.4(4.6)-Dimehtyl-6(2)-isobutyl-dihydro-1.3.5-dithiazin, erhalten durch Beispiel 1, zu A hergestellt.

Eine Aromakomposition D wird durch Zugabe von 5 Gewichtsteilen des Isomerengemisches 2.4(4.6)-Dimethyl-6(2)-(2-butyl)-dihydro-1.3.5-dithiazin, erhalten nach Beispiel 2, zu A hergestellt.

Eine Aromakomposition E wird durch Zugabe von 10 Gewichtsteilen 2.4-Dimethyl-tetrahydro-pyrrolo-[2.1-d]-[1.3.5]-dithiazin, erhalten nach Beispiel 5, zu A hergestellt.

A bis E werden in einer Dosierung von jeweils 10 ppm zu einer Lösung von 5 % Saccharose in Wasser gegeben.

Ein Geschmacksvergleich von B, C, D und E gegen A ergibt:

B: mehr Impact, leichte Popcornnote
C: mehr Impact, etwas nussiger, mehr Röstcharakter
D: mehr Impact, stärkere Röstnote, mehr Nußcharakter
E: mehr Impact, ausgeprägter Röstcharakter

**Beispiel 7**

Ein Schokoladenaroma F wird durch Mischen folgender Bestandteile hergestellt:

| | | |
|---|---|---|
| Isovaleraldehyd | 1-%-ig in Ethanol | 1 |
| Phenylethylalkohol | 0,1-%-ig in Propylenglykol | 1 |
| Vanillin | | 5 |
| Phenylessigsäure | 1-%-ig in Propylenglykol | 5 |
| Ethylvanillin | | 50 |
| Kakaopulverextrakt | 30-%-ig in Propylenglykol | 948 |
| | Gewichtsteile | 1000 |

Durch Zugabe von 0,1 Gewichtsteilen 2.4(4.6)-Dimethyl-6(2)-isopropyl-dihydro-1.3.5-dithiazin zu F wird ein Schokoladenaroma G, durch Zugabe von 0,1 Gewichtsteilen 2.4(4.6)-Dimethyl-6(2)-isobutyl-dihydro-1.3.5-dithiazin zu F wird ein Schokoladenaroma H, durch Zugabe von 0,1 Gewichtsteilen 2.4(4.6)-Dimethyl-6(2)-(2-butyl)-dihydro-1.3.5-dithiazin zu F wird ein Schokoladenaroma I und durch Zugabe von 0,1 Gewichtsteilen 2,4-Dimethyl-tetrahydro-pyrrolo-[2.1-d]-[1.3.5]-dithiazin zu F ein Schokoladenaroma K erhalten.

F bis K werden in einer Dosierung von jeweils 200 ppm zu einer Lösung von 5 % Saccharose in Wasser gegeben. Ein Geschmacksvergleich von G, H, I und K gegen F ergibt:

G: Kakaonote ist ausgeprägter
H: Schokoladennote ist ausgeprägter, etwas an Popcorn erinnernd
I: Dunkle Kakaonote ist stärker
K: Schokoladennote ist stärker, wirkt staubiger

**Beispiel 8**

Ein Erdnußaroma L wird durch Mischen folgender Bestandteile hergestellt:

| | |
|---|---|
| 2-Methyl-3-ethylpyrazin | 5 |
| Phenylacetaldehyd 10-%-ig in Triazin | 5 |
| 2,5-Dimethylpyrazin | 10 |
| Erdnußpaste | 150 |
| Erdnußöl | 830 |
| Gewichtsteile | 1000 |

Durch Zugabe von 0,2 Gewichtsteilen 2.4(4.6)-Dimethyl-6(2)-isopropyl-dihydro-1.3.5-dithiazin zu L wird ein Erdnußaroma M, durch Zugabe von 0,1 Gewichtsteilen 2.4(4.6)-Dimethyl-6(2)-isobutyl-dihydro-1.3.5-dithiazin zu L wird ein Erdnußaroma N, durch Zugabe von 0,1 Gewichtsteilen 2.4(4.6)-Dimethyl-6(2)-(2-butyl)-dihydro-1.3.5-dithiazin zu L wird ein Erdnußaroma O und durch Zugabe von 0,2 Gewichtsteilen 2,4-Dimethyl-tetrahydro-pyrrolo-[2.1-d]-[1.3.5]-dithiazin zu L wird ein Erdnußaroma P erhalten.

L bis P werden in einer Dosierung von 500 ppm zu Milch, die 0,5 % Kochsalz und 0,02 % Mononatriumglutamat enthält, gegeben. Ein Geschmacksvergleich von M, N, O und P gegen L ergibt:

M: mehr Impact, typischer Erdnuß, deutlich stärkerer Röstcharakter
N: mehr Impact, typischer Erdnuß, mehr Röstnote
O: mehr Impact, typischer Erdnuß, wesentlich stärkere Röstnote
P: mehr Impact, typischer Erdnuß, mehr Röstnote

**Beispiel 9**

Durch Zugabe von 0,3 ppm 2.4(4.6)-Dimethyl-6(2)-isopropyl-dihydro-1.3.5-dithiazin zu einer Fleischbrühe wird eine Fleischbrühe Q, durch Zugabe von 0,2 ppm 2.4(4.6)-Dimethyl-6(2)-isobutyl-dihydro-1.3.5-dithiazin eine Fleischbrühe R, durch Zugabe von 0,2 ppm 2.4(4.6)-Dimethyl-6(2)-(2-butyl)-dihydro-1.3.5-dithiazin eine Fleischbrühe S und durch Zugabe von 0,1 ppm 2,4-Dimethyl-tetrahydro-pyrrolo-[2.1-d]-[1.3.5]-dithiazin eine Fleischbrühe T erhalten.

Ein Geschmacksvergleich von Q, R, S und T gegen die unbehandelte Fleischbrühe ergibt:

Q: Die Fleischbrühe bekommt einen volleren und fleischigeren Geschmack in Richtung Bratenfleisch
R: Der Geschmack wird voller und fleischiger in Richtung Bratenfleisch
S: Der Geschmack wird voller und fleischiger
T: Der Geschmack wird voller und fleischiger

**Patentansprüche**

1. Unsymmetrische Dihydro-dithiazine der Formel

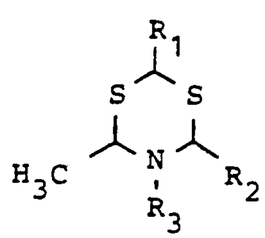

in der

einer der Substituenten $R_1$ und $R_2$ für eine $C_3$-$C_5$-Alkylgruppe steht während der andere eine Methylgruppe ist und $R_3$ für ein Wasserstoffatom steht

oder $R_1$ für eine Methylgruppe steht und $R_2$ und $R_3$ zusammen einen $C_3$-$C_4$-Alkylenrest bilden.

2. Verfahren zur Herstellung unsymmetrischer Dihydrodithiazine der Formel

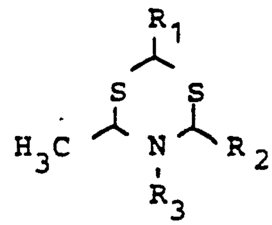

in der

einer der Substituenten $R_1$ und $R_2$ für eine $C_3$-$C_5$-Alkylgruppe steht während der andere eine Methylgruppe ist und $R_3$ für ein Wasserstoffatom steht

oder $R_1$ für eine Methylgruppe steht und $R_2$ und $R_3$ zusammen einen $C_3$-$C_4$-Alkylenrest bilden,

dadurch gekennzeichnet, daß man zur Herstellung der monocyclischen Verbindungen ein Gemisch aus Acetaldehyd und einem $C_4$-$C_6$-Alkanal mit Ammoniak reagieren läßt und das Reaktionsprodukt mit Schwefelwasserstoff umsetzt und zur Herstellung der bicyclischen Verbindungen Acetaldehyd mit 1-Pyrrolin oder 2.3.4.5-Tetrahydropyridin reagieren läßt und das Reaktionsprodukt mit Schwefelwasserstoff umsetzt.

3. Verwendung der unsymmetrischen Dihydro-dithiazine gemäß Anspruch 1 als Riech- und Geschmackstoffe.

**Claims**

1. Unsymmetrical dihydrodithiazines of the formula in which

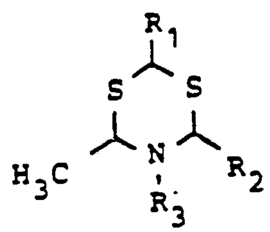

in which

one of the substituents $R_1$ and $R_2$ represents a $C_3$-$C_5$-alkyl group, while the other methyl group, and $R_3$ represents a hydrogen atom,

or $R_1$ represents a methyl-group and $R_2$ and $R_3$ together form a $C_3$-$C_4$-alkylene radical.

2. Process for the preparation of unsymmetrical dihydrodithiazines of the formula

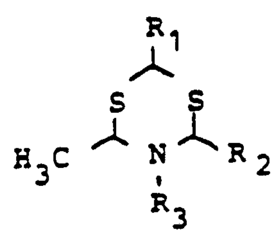

in which

one of the substituents $R_1$ and $R_2$ represents a $C_3$-$C_5$-alkyl group, while the other is a methyl group, and $R_3$ represents a hydrogen atom,

or $R_1$ represents a methyl group and $R_2$ and $R_3$ together form a $C_3$-$C_4$-alkylene radical,

characterised in that the monocyclic compounds are prepared by reacting a mixture of acetaldehyde and a $C_4$-$C_6$-alkanal with ammonia, and reacting the product of the reaction with hydrogen sulphide, and the bicyclic compounds are prepared by reacting acetaldehyde with 1-pyrroline or 2,3,4,5-tetrahydropyridine, and reacting the product of the reaction with hydrogen sulphide.

3. Use of the unsymmetrical dihydrodithiazines according to Claim 1 as fragrances and flavourings.

**Revendications**

1. Dihydro-dithiazines asymétriques, de formule

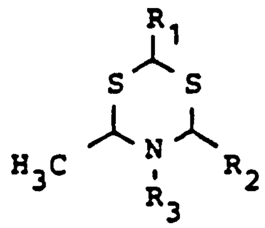

dans laquelle
l'un des substituants $R_1$ et $R_2$ représente un groupe alkyle en $C_3$ à $C_5$, cependant que l'autre représente un groupe méthyle et $R_3$ est un atome d'hydrogène,
ou bien $R_1$ représente un groupe méthyle et $R_2$ et $R_3$ forment ensemble un reste alkylène en $C_3$ ou $C_4$.

2. Procédé pour préparer des dihydro-dithiazines asymétriques de formule:

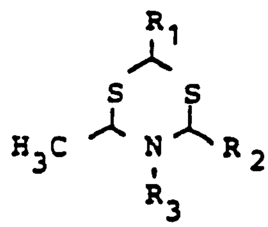

dans laquelle
l'un des substituants $R_1$ et $R_2$ représente un groupe alkyle en $C_3$ à $C_5$ cependant que l'autre substituant représente un groupe méthyle et que $R_3$ est un atome d'hydrogène,
ou bien $R_1$ représente un groupe méthyle et $R_2$ et $R_3$ forment ensemble un reste alkylène en $C_3$ à $C_4$,
procédé caractérisé en ce que, pour préparer les composés monocycliques, on fait réagir un mélange de l'acétaldéhyde et d'un alcanal en $C_4$ à $C_6$ avec de l'ammoniaque et l'on fait réagir le produit de cette réaction avec de l'hydrogène sulfuré et, pour préparer les composés bicycliques, on fait réagir l'acétaldéhyde avec la 1-pyrroline ou la 2,3,4,5-tétrahydropyridine et l'on fait réagir le produit de cette réaction avec de l'hydrogène sulfuré.

3. Utilisation des dihydro-dithiazines asymétriques selon la revendication 1, comme parfums et substances d'aromatisation ou flavorisantes.